# NEUE EUROPÄISCHE PATENTSCHRIFT

(11) **EP 1 492 594 B2**
(45) Veröffentlichungstag und Bekanntmachung des Hinweises auf die Entscheidung über den Einspruch: **04.03.2015**
(45) Hinweis auf die Patenterteilung: 02.06.2010
(21) Anmeldenummer: 03717256.6
(22) Anmeldetag: 01.04.2003
(51) Int. Cl.: A61N 7/00, A61H 23/00

(54) **MEDIZINISCHES GERÄT ZUR BEHANDLUNG VON BIOLOGISCHEM GEWEBE**
MEDICAL DEVICE FOR THE TREATMENT OF BIOLOGICAL TISSUE
INSTRUMENT MEDICAL DESTINE AU TRAITEMENT DE TISSUS BIOLOGIQUES

(30) Priorität: 08.04.2002 DE 10215416
(43) Veröffentlichungstag der Anmeldung: 05.01.2005
(73) Patentinhaber: Ferton Holding SA, 2800 Delémont (CH)
(72) Erfinder: MENNE, Andreas, 1274 Signy (CH); SCHULZ, Manfred, 88662 Überlingen (DE); HAUPT, Gerald, 50169 Kerpen (DE)
(74) Vertreter: Von Kreisler Selting Werner - Partnerschaft von Patentanwälten und Rechtsanwälten mbB
(86) Internationale Anmeldenummer: PCT/EP2003/003373
(87) Internationale Veröffentlichungsnummer: WO 2003/084608

(56) Entgegenhaltungen:
- DE-A- 19 725 477
- DE-A1- 3 506 583
- DE-A1- 19 929 112
- DE-C2- 3 814 743
- US-A- 3 499 437
- US-A- 4 315 514
- US-A- 4 513 737
- US-A- 5 160 336
- US-A- 5 727 556
- US-A1- 2001 014 780
- 'Extrakorporale Stimulation des Herzens durch Druckpulse' DORNIER MEDIZINETECHNIK
- DR. OTHMAR WESS, ET AL.: 'Externe Herzstimulation durch Druckpulse' DORNIER POST 01 Januar 1981,
- L.D. ROZENBERG: 'Transmission of ultrasonic vibrations into media to be processed' ULTRASONIC TECHNOLOGY Bd. 2, 22 Mai 1969, NEW-YORK, Seiten 3 - 58
- FRANK S. CRAWFORD, JR.: 'Schwingungen und Wellen' BERKELEY PHYSIK KURS 3 Bd. 1982, Seiten 106 - 107

## Beschreibung

Die Erfindung betrifft ein medizinisches Instrument zur Behandlung von biologischem Gewebe nach dem Oberbegriff des Anspruchs 1.

Derartige Instrumente dienen dazu, mittels Druck- oder Stoßwellen den Heilungsprozeß bei Knochenbrüchen und Knochendefekten, aber auch bei der Parodontose zu beschleunigen oder überhaupt erst in Gang zu bringen. Weitere Einsatzgebiete sind die Behandlung von chronischen Schmerzen bei Sehnenansatzerkrankungen und die Auflösung von myofaszialen Trigger-Arealen. Es wird vermutet, daß mit Hilfe der Druckwellen Mikroschädigungen im biologischen Gewebe erzeugt werden, die den Körper zu Regenerationsmaßnahmen veranlassen.

Für solche Anwendungen werden bislang sogenannte extrakorporale Druck- oder Stoßwellengeräte benutzt. Diese Geräte erzeugen einen akustischen Impuls und leiten ihn über die Hautoberfläche auf das Zielgebiet innerhalb des Körpers weiter, wo er seine Wirkung dann entfaltet. Ein einfach aufgebautes Gerät dieser Geräteklasse ist in der Patentschrift DE-A-197 25 477 beschrieben. Hier wird der akustische Impuls durch den Aufschlag eines Projektils erzeugt und unfokussiert über ein stumpfes Übertragungselement in den Körper eingekoppelt. Andere solcher medizinischen Geräte fokussieren den akustischen Impuls auf das Zielgebiet. Ein typisches Beispiel für ein solches Gerät ist in der Deutschen Offenlegungsschrift DE-A-23 51 247 zu finden. Hier dient eine Funkenentladung als Quelle für den akustischen Impuls, welcher durch einen ellipsoidförmigen Reflektor fokussiert wird. Zur Generierung der Druckwellen gehören mittlerweile auch elektromagnetische und piezoelektrische Quellen (DE-a-35 02 751) zum Stand der Technik. Bekannte alternative Mittel zur Fokussierung sind der Einsatz akustische Flüssigkeits- oder Feststofflinsen (US-A-5 727 875), die Ausbildung der akustischen Quelle als bewegte Kugelkalottenfläche (DE-C-33 12 014) oder auch die Anordnung mehrerer Quellen auf einer Kugeloberfläche (DE-A-199 28 491), wie sie bei piezoelektrischen Antrieben häufig zum Einsatz kommen.

Zum Betrieb aller bekannten fokussierenden Systemen ist ein Hochspannungsnetzteil notwendig, um die kurzen, aber heftigen, akustischen Impulse zu generieren. Dies macht die Geräte aufwendig, limitiert die maximale Wiederholfrequenz pro Zeiteinheit bei noch sinnvoller Baugröße und erfordert Sicherheitsmaßnahmen zur Isolierung der Hochspannung. Mit Ausführungsformen gemäß der DE-A-197 25 477 ist eine Fokussierung der akustischen Energie nicht möglich und Anwendungen demnach auf oberflächennahe Indikationen beschränkt.

Aus der US 2001/0014780 ist ein medizinisches Instrument bekannt, das mit Hilfe eines Ultraschallwandlers, der eine nach innen gewölbte konkave Austrittsgrenzfläche aufweist, eine fokussierte Druckwelle über ein Impedanzanpassungsmittel eingekoppelt. Die Erzeugung und Fokussierung der Druckwellen erfolgt dabei nicht an der Austrittsgrenzfläche des Impedanzanpassungsmittels.

Aus der US-A-5 160 336 ist ein ballistischer Druckwellengenerator bekannt, wie er auch grundsätzlich aus der DE-A-197 25 477 bekannt ist.

Aus der US-A-4 315 514 ist ein Verfahren zum selektiven Zerstören von Zellen bekannt, bei dem Ultraschallwellen mit einer Resonanzfrequenz der zu zerstörenden Zellen betrieben werden und mit Hilfe einer aus Polystyren gebildeten Linse auf die zu zerstörenden Zellen fokussiert werden.

Der Erfindung liegt demzufolge die Aufgabe zugrunde, ein Druck- oder Stoßwellengerät so auszubilden, daß es auf einfache und kostengünstige Weise Druck- oder Stoßwellen erzeugt und diese auf ein Zielgebiet im Körper fokussiert. Zur Lösung dieser Aufgabe dienen die Merkmale des Anspruchs 1.

Die Erfindung besteht in vorteilhafter Weise aus einem primären Druckwellengenerator, bei dem ein Schlagteil mit Hilfe eines Antriebsmittels auf eine hohe Endgeschwindigkeit beschleunigbar ist und auf ein Übertragungselement einen Kraftstoß ausübt. Infolge dieses Kraftstoßes induziert das Schlagteil eine Druckwelle in dem Übertragungselement, welche sich in diesem in Richtung seiner Austrittsgrenzfläche fortpflanzt, um von dort in das biologische Gewebe eingekoppelt zu werden. Die Austrittsgrenzfläche ist dabei so beschaffen, daß die austretende Welle in dem biologischen Gewebe einen Fokus bildet. Dies wird durch eine Formgebung des Übertragungselementes erreicht, bei der für jede lokale Einzelwelle die Laufzeit von der Erzeugung beim Kraftstoß bis zum Fokus gleich ist. Die Eintrittsgrenzfläche des Übertragungselementes ist dabei kleiner als die Austrittsgrenzfläche. In erster Näherung würde sich für ein gerades Übertragungselement aus Stahl eine nahezu kugelförmige Austrittsgrenzfläche ergeben. Die Austrittsgrenzfläche hat einen größeren Durchmesser als die Eintrittsgrenzfläche oder die Querabmessung des Schlagteils.

Das Übertragungselement ist als Exponentialtrichter ausgebildet, welcher die ebene Welle verlustfrei von kleinen auf größere Querschnitte überführt.

Als Schlagteil ist vorzugsweise ein aus Metall oder einem anderen hochfesten Material bestehendes Projektil vorgesehen, welches in einer Führung mittels eines Druckluftpulses hin- und herbewegbar ist. Der pneumatische Antrieb eignet sich aufgrund seiner Einfachheit und des Gewichtsvorteils besonders gut, um das Schlagteil auf eine hohe Endgeschwindigkeit und damit verbundenen hohen Impulsenergien zu beschleunigen. Aber auch andere Antriebe durch einen Federmechanismus oder auf elektromagnetische Weise sind denkbar.

Insbesondere bei orthopädischen Anwendungen ist es vorteilhaft, eine Vielzahl von einzelnen Impulsen in das biologische Gewebe einzukoppeln, um eine optimale Wirkung zu erzielen. Das Antriebsmittel ist daher vorzugsweise so ausgestaltet, daß eine periodische Hin- und Herbewegung des Schlagteils möglich ist. Die Schlagzahl beträgt ca. 1 bis 30 Hz, vorzugsweise 5 bis 12 Hz. Bei einer für orthopädische Anwendungen zur Zeit empfohlenen Impulszahl von ca. 2000 pro Sitzung sind damit Behandlungszeiten von unter fünf Minuten möglich.

In einer bevorzugten Ausführungsform ist das Übertragungselement axial und linear in einem Gehäuse geführt, wobei ein Feder-/Dämpfungselement zwischen dem Übertragungselement und dem Gehäuse angeordnet ist. Auf diese Weise wird eine Entkopplung des Übertragungselementes realisiert. Außerdem ist eine große Auslenkung des Übertragungselementes nicht notwendig und auch nicht gewünscht, da diese von den Patienten als besonders schmerzhaft empfunden wird. Vielmehr erfolgt die Wellenübertragung durch eine Kompression bzw. Expansion des Übertragungselementes und nicht durch seine Verlagerung. Typische Werte für den Hub des Übertragungselementes liegen daher auch bei weniger als 0,5 mm.

Zwischen dem Schlagteil und dem Übertragungselement kann ein Zwischenelement angeordnet sein, daß den Kraftstoß von dem Schlagteil auf das Übertragungselement weiterleitet. Diese Zwischenstück kann dazu dienen, eine bessere Abschirmung der Antriebsmittel gegenüber dem Applikationsbereich zu schaffen oder auch zum Umlenken der Richtung der Druckwelle oder aber zum Beeinflussen der Druckwellencharakteristik.

Das Übertragungselement besteht vorzugsweise aus einem hochfesten Material, wie z.B. Stahl, um der Belastung durch das einwirkende Schlagteil Stand zu halten. In einer bevorzugten Ausführungsform sind die schlagende Fläche des Schlagteils und die getroffene Oberfläche des Übertragungselementes eben und senkrecht zur Bewegungsrichtung des Schlagteils. In einem solchen Fall entsteht durch den Aufschlag eine ebene Welle in dem Übertragungselement, welche sich in diesem fortpflanzt. Besteht das Übertragungselement aus einem Bolzen ohne wesentliche Querschnittsänderung, so behält die Welle ihre Form bei und wandert als ebene Welle in Richtung ihrer Austrittsgrenzfläche. Die bevorzugte Austrittsgrenzfläche des Übertragungselementes hat nahezu die Form einer nach innen gewölbten Kugeloberfläche. In einem solchen Fall gibt es einen Punkt in dem biologischen Gewebe, in welchem alle einzelnen lokalen Wellen - bedingt durch die unterschiedlichen Schallgeschwindigkeiten im Übertragungselement und im biologischen Gewebe - zur gleichen Zeit eintreffen und damit einen Fokus bilden. Die Position des Fokus im biologischen Gewebe relativ zum Übertragungselement kann durch die Auswahl des Krümmungsradiuses der Austrittsgrenzfläche voreingestellt werden. Die ideale Geometrie der Austrittsgrenzfläche weicht etwas von einer Kugeloberfläche ab und kann rechnerisch bestimmt werden.

Zur Steigerung der abgestrahlten akustischen Leistung des Übertragungselementes ist seine Austrittsgrenzfläche möglichst groß zu wählen, der Durchmesser des Schlagteils aber möglichst klein zu halten, um die bewegten Massen und Impulse für eine medizinische Anwendung handhabbar zu gestalten. Es hat sich gezeigt, daß bei gleichem Durchmesser von Schlagteil und dem Austrittsbereich des Übertragungselementes eine Fokussierung nur bedingt möglich ist.

Für eine optimale Erzeugung der Druckwelle im Übertragungselement sind die schlagende Fläche des Schlagteils und die Eintrittsfläche des Übertragungselementes gleich groß zu wählen. Der Austrittsdurchmesser des Übertragungselementes ist größer als die Querabmessung des Schlagteils, bzw. sein eigener Eintrittsdurchmesser zu bemaßen.

Unter Vernachlässigung der idealen Weiterleitung sind auch beliebige andere Querschnittserweiterung des Übertragungselementes von seinem Eintritts- hin zum Austrittsdurchmesser denkbar. Die Geometrie der Austrittsgrenzfläche des Übertragungselementes ist dann wiederum so zu wählen, daß die Laufzeiten der Welle einen Fokus im biologischen Gewebe bilden.

Bei solchen Übertragungselementen stehen die äußeren Kanten vor, welche durch die nach innen gewölbte Austrittsgrenzfläche gebildeten werden. Da durch den Aufschlagimpuls eine Bewegung des Übertragungselementes nicht vollständig vermieden werden kann, können diese vorstehenden Kanten das biologische Gewebe schädigen oder zumindest reizen. Daher sind bei einer bevorzugten Ausführungsform diese äußeren Kanten atraumatisch ausgebildet. Ein Abrunden der Kanten oder ein schützender Überzug sind geeignete Maßnahmen. Ebenso ist es vorstellbar, daß die äußeren Kanten des Gehäuses leicht in axiale Richtung überstehen, so daß das Übertragungselement nicht in direkten Kontakt mit dem biologischen Gewebe kommt.

Zwischen der Austrittsgrenzfläche des Übertragungselementes und der Einkoppelstelle auf dem biologischen Gewebe kann ein Impedanzanpassungsmedium angeordnet sein, das die Einkopplung der Druckwelle in das biologische Gewebe verbessert. Befinden sich zwischen der Austrittsgrenzfläche des Übertragungselementes und der Eintrittsfläche des biologischen Gewebes Lufteinschlüsse, so wird ein Teil des Druckimpulses an dieser akustischen Unstetigkeit reflektiert und der übertragene Anteil gemindert. Ein geeignetes pastenförmiges Impedanzanpassungsmedium ist beispielsweise ein Ultraschallgel oder andere pastenfömige Massen mit einer ähnlichen Impedanz wie das biologische Gewebe (z.B. Vaseline).

Zur Vermeidung von Lufteinschlüssen kann auch der durch die Kugelfläche gebildete Hohlraum mit einem festen, akustisch gut leitendem Material ausgekleidet sein. Ein solches geeignetes Material ist z.B. Polystyrol.

Es zeigen:
**Fig.** 1 und 2 Darstellungen eines mechanisch betriebenen medizinischen Instruments im Querschnitt, und
Fig. 3 bis 6 eine Reihe von verschiedenen Ausbildungen eines Übertragungselementes.

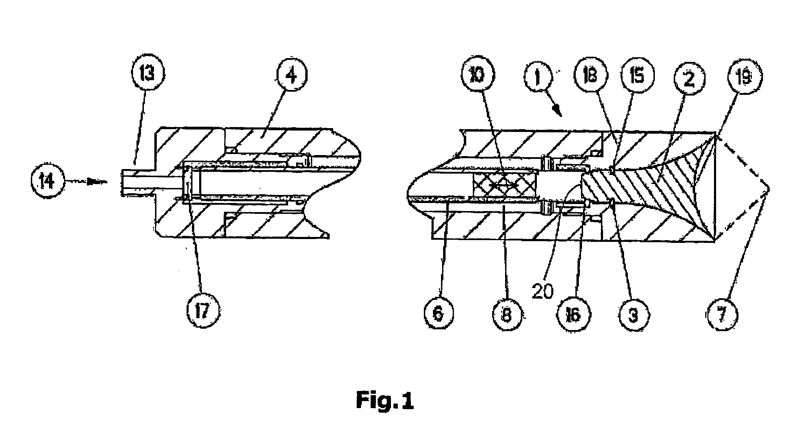

Das in **Fig.** 1 gezeigte Handstück **1** besteht aus einem Gehäuse **4,** das einen pneumatischen Innenzylinder **6** aufnimmt, in dem ein Schlagteil **10** mit Hilfe pneumatischer Antriebsmittel **14** in Verbindung mit einer Staudruckkammer **8,** die den Innenzylinder **6** koaxial ringförmig umgibt, zwischen zwei Endpositionen hin und her bewegt wird. Alternativ ist es auch möglich das Schlagteil **10** hydraulisch, mechanisch, elektromagnetisch oder durch andere Antriebsmittel zu bewegen. Je nach Antriebsart kann eine geeignete Länge des Beschleunigungswegs ausgewählt werden. Bei einem pneumatisch betriebenen Schlagwerk und einem gebräuchlichen Pressluftdruck von ca. 0,3 MPa (3 bar) beträgt der Beschleunigungsweg etwa 50 bis 200 mm. Das Schlagteil **10** kann zwecks Charakterisierung der Druck- oder Stoßwelle in seiner Länge, Endgeschwindigkeit und Materialzusammensetzung ausgewählt werden. Eine Leistungseinstellung des medizinischen Instrumentes erfolgt üblicherweise durch eine Regulierung der Druckluft mittels eines Druckminderers.

In der proximalen Endposition des Schlagteils **10** ist am Ende des Innenzylinders **6** ein Magnethalter **17** angeordnet, der das metallische Schlagteil **10** in seiner proximalen Endposition festhalten kann bis erneut ein über den Anschluß **13** aufgebrachter pneumatischer Druck das Schlagteil **10** in Richtung auf das distale Ende des Innenzylinders **6** beschleunigt. Die sich in Bewegungsrichtung des Schlagteils **10** befindliche Luft wird über an einen an dem distalen Ende des Innenzylinders **6** befindlichen Ringschlitze **16** in die Staudruckkammer **8** geleitet. Durch die Beschleunigung des Schlagteils **10** trifft dieses mit hoher Endgeschwindigkeit von beispielsweise 10 bis 25 m/s auf die distal von dem Innenzylinder **6** angeordnete Eintrittsgrenzfläche 20 eines Übertragungselementes **2** und induziert in diesem eine Druck- oder Stoßwelle, die sich bis zu seiner Austrittsgrenzfläche **19** fortpflanzt und dann in das biologische Gewebe eingekoppelt wird. Das Übertragungselement **2** besteht aus einem metallischen Material und ist in einer Aufnahme **18** gleitend geführt. Eine Ringnut **3** ist im Übertragungselement **2** und in der Aufnahme **18** angeordnet, in welcher sich ein elastisches Feder-/Dämpfungselement **15** befindet. Dieses hat die Aufgabe das Übertragungselement **2** von der Aufnahme **18** zu entkoppeln, sorgt aber auch dafür, daß das Übertragungselement **2** nach dem Schlagvorgang wieder in seine initiale Position zurückkehrt. Gleichzeitig dichtet das Feder-/Dämpfungselement **15** die Druckkammer **8** gegen den Außenraum ab und verhindert damit ein Austreten der Druckluft und das Eindringen von Schmutz. Die Austrittsgrenzfläche **19** des Übertragungselementes **2** ist als Teil einer Kugeloberfläche ausgebildet. Der Fokus **7** der austretenden Druck- oder Stoßwelle entspricht in erster Näherung dem geometrischen Mittelpunkt der Kugeloberfläche.

Nach Beendigung des Schlagvorgangs bewegt das Feder-/Dämpfungselement **15** das Übertragungselement **2** wieder in seine Ausgangsposition zurück. Das Schlagteil **10** wird durch den in der Staudruckkammer **8** aufgebauten Überdruck durch das Zurückströmen der Luft durch die Ringschlitze **16** in seine Ruheposition am proximalen Ende des Innenzylinders **6** zurückgeführt und von dem Magnethalter **17** fixiert. Das Instrument ist nun wieder zu einem erneuten Schlagvorgang bereit.

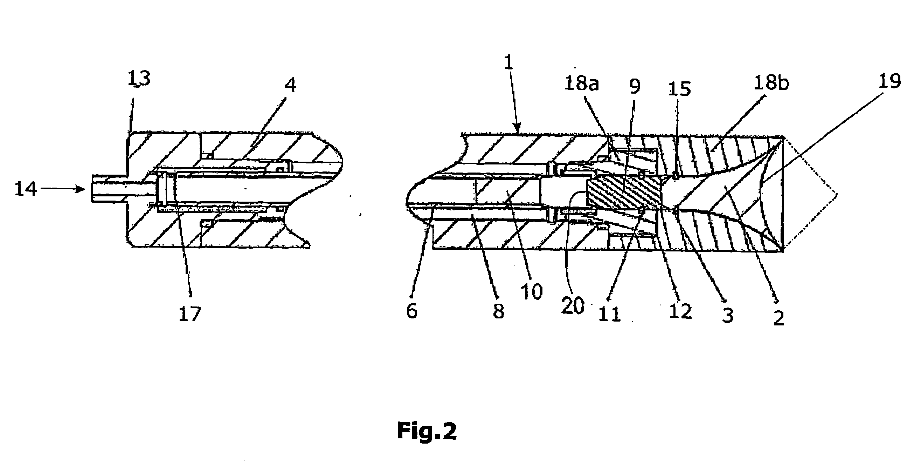

Die in **Fig. 2** dargestellte Ausführungsform besitzt zusätzlich ein Zwischenstück **9** mit einer Abdichtung **11,** welches zwischen Schlagteil **10** und Übertragungselement **2** angeordnet ist. Dieses Bauteil hat die Aufgabe von dem Schlagteil **10** getroffen zu werden und den Schlagimpuls auf das Übertragungselement **2** weiterzuleiten. Die Aufnahme **18a** und **18b** ist hier zweiteilig aufgebaut. Durch Lösen der Drehverbindung 12 kann das Übertragungselement **2** und die vordere Aufnahme **18a** entfernt werden. Das Handstück **1** bleibt dabei geschlossen und kann einfach gereinigt, desinfiziert und sterilisiert werden, ohne daß Flüssigkeit oder Schmutz in das Innere des Handstückes **1** eindringen könnte.

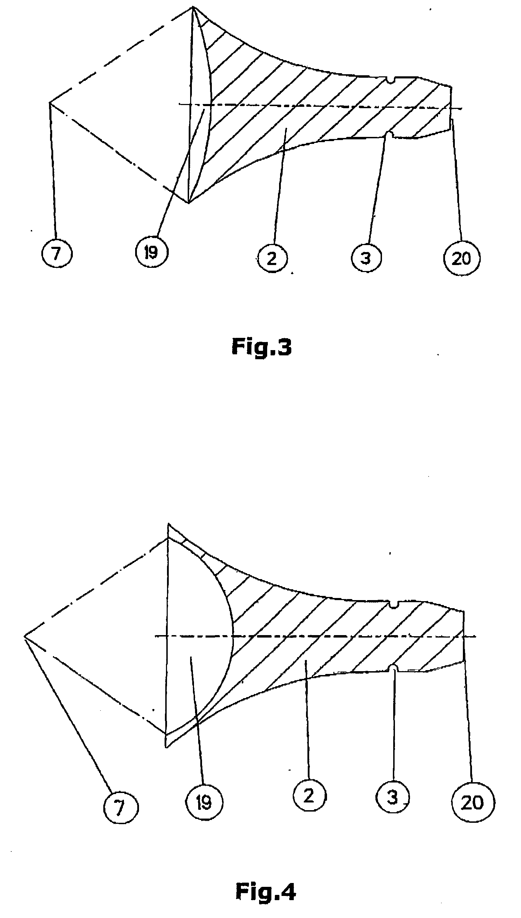

Durch Variation des Krümmungsradius der Austrittsgrenzfläche **19** des Übertragungselementes **2** kann die Position des Fokus **7** eingestellt werden. In **Fig. 3** ist ein Übertragungselement **2** gezeigt, welches einen großen Krümmungsradius aufweist, wodurch der Fokus **7** weiter hinter der Austrittsgrenzfläche **19** zu liegen kommt. **Fig. 4** zeigt eine Ausführungsform mit einem kleinen Krümmungsradius.

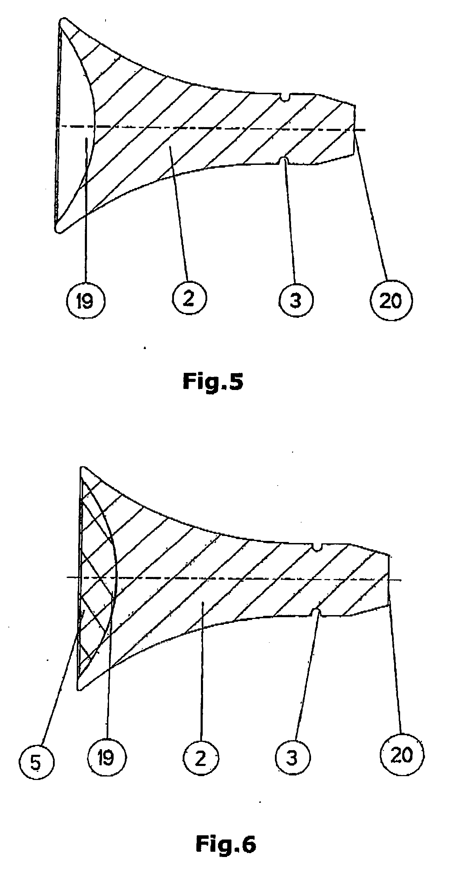

Bedingt durch die geometrische Form der Austrittsgrenzfläche **19** des Übertragungselementes **2** bilden sich scharfe Kanten, die zu einer Verletzung oder Reizung des biologischen Gewebes führen können. Aus diesem Grund sind die äußeren Kanten des Übertragungselementes **2** in **Fig. 5** abgerundet.

Um Lufteinschlüsse zu vermeiden und eine ebenen Oberfläche des Übertragungselementes **2** zu erhalten, ist die kugelförmige Austrittsgrenzfläche **19** des Übertragungselementes **2** in **Fig. 6** mit einem akustisch gut leitendem Einsatz **5** ausgekleidet, welches ähnliche Impedanzen wie das biologische Gewebe besitzt

## Patentansprüche

1. Medizinisches Instrument zur Behandlung von biologischem Gewebe, mit einer Einrichtung zum Erzeugen von extrakorporalen Druckwellen mit einer nach innen gewölbten Austrittsgrenzfläche (19) für Druckwellen, welche derart ausgebildet ist, dass die Druckwellen in das biologische Gewebe einkoppelbar sind und in dem biologischen Gewebe fokussierbar sind,
worin die Einrichtung zum Erzeugen von extrakorporalen Druckwellen ein Übertragungselement (2) zum Einkoppeln der Druckwellen in den Körper von Lebewesen aufweist,
worin die Austrittsgrenzfläche (19) an dem Übertragungselement (2) ausgebildet ist,
worin sich die Druckwellen in dem Übertragungselement (2) bis zur Austrittsgrenzfläche (19) fortpflanzen und durch das Übertragungselement (2) an der Austrittsgrenzfläche (19) fokussierbar sind,
**dadurch gekennzeichnet,**
**dass** die Druckwellen durch das Auftreffen eines Schlagteils (10) auf eine Eintrittsgrenzfläche (20) des Übertragungselementes (2) erzeugbar sind,
**dass** das Übertragungselement an der Austrittsgrenzfläche (19) einen größeren Durchmesser aufweist als an der Eintrittsgrenzfläche (20) oder als die Querabmessung des Schlagteils (10), und
**dass** das Übertragungselement (2) die Form eines Exponentialtrichters aufweist.

2. Medizinisches Instrument nach Anspruch 1, **dadurch gekennzeichnet, dass** die Einrichtung zum Erzeugen der Druckwellen aus einem in einem Gehäuse geführten mit Hilfe eines Antriebsmittels hin- und herbewegbaren Schlagteil (10) besteht, das auf das Übertragungselement (2) einen oder mehrere Kraftstöße ausübt, wobei das Schlagteil (10) infolge des Kraftstoßes eine Druckwelle in dem Übertragungselement (2) induziert, die sich bis zu der Austrittsgrenzfläche (19) des Übertragungselementes (2) fortpflanzt.

3. Medizinisches Instrument nach Anspruch 2, **dadurch gekennzeichnet, dass** das Schlagteil (10) koaxial zu dem Übertragungselement (2) angeordnet ist.

4. Medizinisches Instrument nach einem der Ansprüche 1 bis 3, **dadurch gekennzeichnet, dass** die Druckwellenquelle periodisch antreibbar ist, wobei das Schlagteil (10) und das Übertragungselement (2) selbsttätig rückstellbar sind.

5. Medizinisches Instrument nach einem der Ansprüche 1 bis 4, **dadurch gekennzeichnet, dass** die Schlagfrequenz des Schlagteils (10) ca. 1 bis 30 Hz, vorzugsweise 1 bis 12 Hz beträgt.

6. Medizinisches Instrument nach einem der Ansprüche 1 bis 5, **dadurch gekennzeichnet, dass** zwischen Übertragungselement (2) und dem Gehäuse (4) ein Feder-/Dämpfungselement (15) angeordnet ist.

7. Medizinisches Instrument nach einem der Ansprüche 1 bis 6, **dadurch gekennzeichnet, dass** die Austrittsgrenzfläche (19) des Übertragung selementes (2) aufgrund des Kraftstoßes einen Hub von weniger als 0,5 mm ausführt.

8. Medizinisches Instrument nach einem der Ansprüche 1 bis 7, **dadurch gekennzeichnet, dass** zwischen dem Schlagteil (10) und dem Übertragungselement (2) ein Zwischenelement (9) angeordnet ist, das den Kraftstoß von dem Schlagteil (10) auf das Übertragungselement (2) weiterleitet.

9. Medizinisches Instrument nach einem der Ansprüche 1 bis 8, **dadurch gekennzeichnet, dass** die Außenkanten der Austrittsgrenzfläche des Übertragungselementes abgerundet sind oder mit einem schützenden Überzug versehen sind.

10. Medizinisches Instrument nach einem der Ansprüche 1 bis 9, **dadurch gekennzeichnet, dass** zwischen der Austrittsgrenzfläche (19) des Übertragungselementes (2) und dem biologischen Gewebe impedanzanpassende Medien (5) angeordnet sind, die das Einkoppeln der Druckwelle in das biologische Gewebe verbessern.

## Claims

1. Medical instrument for the treatment of biological tissue, comprising a means for generating extracorporeal pressure waves and having an inwardly curved exit boundary surface (19) for pressure waves configured such that the pressure waves may be coupled into the biological tissue and may be focussed in the biological tissue,
the means for generating extracorporeal pressure waves comprising a transmission element (2) for coupling the pressure waves into the bodies of living beings,
the exit boundary surface (19) being formed on the transmission element (2),
the pressure waves propagating in the transmission element (2) up to the exit boundary surface (19) and being focusable on the exit boundary surface (19) by the transmission element (2),
**characterized in that**
the pressure waves may be generated by an impact member (10) hitting an entry boundary surface (20) of the transmission element (2), and
the transmission element has a diameter at the exit boundary surface (19) that is larger than the diameter at the entry boundary surface (20) or the transverse dimension of the impact member (10), and
the transmission element (2) is in the shape of an exponential horn.

2. Medical instrument of claim 1, **characterized in that** the means for generating the pressure waves is an impact member (10) guided in a housing and adapted to be reciprocated by means of a drive means, the impact member (10) exerting one or more impulses on the transmission element (2) and inducing a pressure wave in the transmission element (2) due to the impulse, said pressure wave propagating to the exit boundary surface (19) of the transmission element (2).

3. Medical instrument of claim 2, **characterized in that** the impact member (10) is arranged coaxially to the transmission element (2).

4. Medical instrument of one of claims 1 to 3, **characterized in that** the pressure wave source may be driven periodically, the impact member (10) and the transmission element (2) being self-returnable.

5. Medical instrument of one of claims 1 to 4, **characterized in that** the impact frequency of the impact member (10) is about 1 to 30 Hz, preferably 1 to 12 Hz.

6. Medical instrument of one of claims 1 to 5, **characterized in that** a spring/damping element (15) is provided between the transmission element (2) and the housing (4).

7. Medical instrument of one of claims 1 to 6, **characterized in that** the exit boundary surface (19) of the transmission element (2) travels a stroke of less than 0.5 mm due to the impulse.

8. Medical instrument of one of claims 1 to 7, **characterized in that** an intermediate element (9) is arranged between the impact member (10) and the transmission element (2), which intermediate element passes the impulse from the impact member (10) to the transmission element (2).

9. Medical instrument of one of claims 1 to 8, **characterized in that** the outer edges of the exit boundary surface of the transmission element are rounded or provided with a protective coating.

10. Medical instrument of one of claims 1 to 9, **characterized in that** impedance-adjusting media (5) are provided between the exit boundary surface (19) of the transmission element (2) and the biological tissue for improving the coupling of the pressure wave into the biological tissue.

## Revendications

1. Instrument médical pour le traitement d'un tissu biologique, comprenant un dispositif servant la production d'ondes de pression extracorporelles et comportant une surface limite de sortie (19) bombée vers l'intérieur, pour des ondes de pression, surface limite de sortie qui est configurée de manière telle, que les ondes de pression puissent être couplées dans le tissu biologique et puissent être focalisées dans le tissu biologique,
dans lequel le dispositif servant à la production d'ondes de pression extracorporelles présente un élément de transmission (2) servant au couplage des ondes de pression dans le corps d'un être vivant,
dans lequel la surface limite de sortie (19) est configurée sur l'élément de transmission (2),
dans lequel les ondes de pression se propagent dans l'élément de transmission (2), jusqu'à la surface limite de sortie (19), et peuvent être focalisées sur la surface limite de sortie (19), par l'élément de transmission (2),
**caractérisé en ce que** les ondes de pression sont produites par l'impact d'une pièce à percussion (10) sur une surface limite d'entrée (20) de l'élément de transmission (2), et
**en ce que** l'élément de transmission présente, sur la surface limite de sortie (19), un diamètre plus grand que celui sur la surface limite d'entrée (20) ou plus grand que la dimension transversale de la piéce à percussion (10), et
**en ce que** l'élément de transmission (2) présente la forme d'un pavillon acoustique exponentiel.

2. Instrument médical selon la revendication 1, **caractérisé en ce que** le dispositif servant à la production d'ondes de pression se compose d'une pièce à percussion (10) guidée dans un carter et pouvant se déplacer en va-et-vient à l'aide d'un moyen d'entrainement, pièce à percussion qui exerce sur l'élément de transmission (2) un ou plusieurs chocs dynamiques, la pièce à percussion (10), suite au choc dynamique, induisant dans l'élément de transmission (2) une onde de pression qui se propage jusqu'à la surface limite de sortie (19) de l'élément de transmission (2).

3. Instrument médical selon la revendication 2, **caractérisé en ce que** la pièce à percussion (10) est disposée de façon coaxiale par rapport à l'élément de transmission (2).

4. Instrument médical selon l'une des revendications 1 à 3, **caracterise en ce que** la source d'ondes de pression peut être déclenchée périodiquement, la pièce à percussion (10) et l'élément de transmission (2) pouvant revenir automatiquement à leur position initiale.

5. Instrument médical selon l'une des revendications 1 à 4, **caractérisé en ce que** la fréquence de percussion de la pièce à percussion (10) est comprise à peu près entre 1 Hz et 30 Hz, de préférence entre 1 Hz et 12 Hz.

6. Instrument médical selon l'une des revendications 1 à 5, **caractérisé en ce qu'**un élément à ressort/d'amortissement (15) est disposé entre l'élément de transmission (2) et le carter (4).

7. Instrument médical selon l'une des revendications 1 à 6, **caractérisé en ce que** la surface limite de sortie (19) de l'élément de transmission (2) exécute, en raison du choc dynamique, une course de moins de 0,5 mm.

8. Instrument médical selon l'une des revendications 1 à 7, **caractérisé en ce qu'**un élément intermédiaire (9) est disposé entre la pièce à percussion (10) et l'élément de transmission (2), élément intermédiaire qui transmet le choc dynamique, depuis la pièce à percussion (10) jusqu'à l'élément de transmission (2).

9. Instrument médical selon l'une des revendications 1 à 8, **caractérisé en ce que** les bords extérieurs de la surface limite de sortie de l'élément de transmission sont arrondis ou sont dotés d'un revêtement de protection.

10. Instrument médical selon l'une des revendications 1 à 9, **caractérisé en ce que** des moyens (5) d'adaptation d'impédance sont disposés entre la surface limite de sortie (19) de l'élément de transmission (2), et le tissu biologique, moyens qui améliorent le couplage de l'onde de pression dans le tissu biologique.
